**Europäisches Patentamt**

**European Patent Office**

**Office européen des brevets**

(19)

(11) Veröffentlichungsnummer: **0 225 479**

**A2**

(12) **EUROPÄISCHE PATENTANMELDUNG**

(21) Anmeldenummer: 86115232.0

(22) Anmeldetag: 04.11.86

(51) Int. Cl.4: **C12N 1/14** , C12M 1/16 , C12M 1/00 , C12N 9/00

(30) Priorität: 11.11.85 DE 3539875

(43) Veröffentlichungstag der Anmeldung:
16.06.87 Patentblatt 87/25

(84) Benannte Vertragsstaaten:
**BE DE FR GB IT NL**

(71) Anmelder: **Keller & Bohacek GmbH & Co KG**
**Liliencronstrasse 64**
**D-4000 Düsseldorf-Rath(DE)**

(72) Erfinder: **Caro, Thomas, Dipl.-Ing.**
**Georgstrasse 24**
**D-5100 Aachen(DE)**

(74) Vertreter: **Gille, Christian, Dipl.-Ing. et al**
**Türk, Gille + Hrabal Patentanwälte Bruckner**
**Strasse 20**
**D-4000 Düsseldorf 13(DE)**

(54) Verfahren und Vorrichtung zum Herstellen enzymhaltiger Biomasse aus Zuckerrübenschnitzeln.

(57) Beschrieben wird ein Verfahren zur Herstellung von enzymhaltiger Biomasse, die zur Gewinnung von Enzymen und Biogas geeignet ist, bei der ausgelaugte Zuckerrübenschnitzel mit Aspergillus niger, insbesondere Aspergillus niger Z 317, fermentiert werden.

Beschrieben wird auch eine Vorrichtung zur Durchführung des Verfahrens, die aus einem vertikalen Behälter besteht, der durch horizontale Trennböden in eine obere erste Anwachszone, eine mittlere zweite Anwachszone und eine untere Rührzone unterteilt ist. Nach der ersten Anwachszone werden bewachsene Zuckerrübenschnitzel als Impfmaterial für das Beschickungsgut entnommen.

FIG.1

EP 0 225 479 A2

## Verfahren und Vorrichtung zum Herstellen enzymhaltiger Biomasse aus Zuckerübenschnitzeln

Bei der Zuckerherstellung fallen in den Zuckerfabriken große Mengen an Zuckerrübenschnitzeln ab. Diese werden zur Zeit etwa in getrocknetem Zustand oder als Preßlinge zur Viehfütterung abgegeben. In der Zukunft ist es jedoch möglich, daß die Milch-und Rindfleischproduktion aus Überschußgründen stark gesenkt werden muß. Infolgedessen kann es Absatzschwierigkeiten für Zuckerrübenschnitzel führen, und es erhebt sich die Frage nach einer anderen wirtschaftlichen Ausnutzung der anfallenden Zuckerrübenschnitzel.

Eine mikrobiologische Verwertung der in großem Maße anfallenden Zuckerrübenschnitzel wurde bisher nicht in Betracht gezogen. Lediglich die Abwässer der Zuckerrübenindustrie wurden mikrobiologisch zur Herstellung von Methan verwertet.

Die DE-OS 28 38 635 beschreibt die Kultivierung von Mikroorganismen auf stückförmigen Kulturmedien. Dabei handelt es sich jedoch um befeuchtete Weizen-bzw. Reiskleien. Die mikrobiologische Verwertung von Zuckerrübenschnitzeln wird nicht beschrieben.

Die DE-OS 29 46 646 beschreibt die Verwertung von Schweinemist als stückförmiges Substrat durch mikrobiologische Behandlung zur Erzielung eines geruchfreien Produkts, das als Futtermittel verwendet werden kann. Auch hier wird die mikrobiologische Verwertung von Zuckerrübenschnitzeln nicht beschrieben.

In beiden Offenlegungsschriften erfolgt die Kultur der Mikroorganismen in vertikalen zylindrischen Behältern mit horizontalen Zwischenwänden. Bei der DE-OS 28 38 635 sind die Zwischenwandelemente in Form von teilweise überlappenden Kreissektoren mit einer Perforation ausgebildet. Die horizontalen Zwischenwände des Fermentationsbehälters der DE-OS 29 46 646 lassen einen ringförmigen Zwischenraum zur Innenwandung des Behälters frei, der einen ringförmigen Kanal für den Durchgang der zu kultivierenden Masse bildet.

Aufgabe der Erfindung ist die Bereitstellung eines Verfahrens und einer Vorrichtung zur Verwertung von frisch extrahierten Zuckerrübenschnitzeln und ähnlichen stückförmigen pflanzlichen Produkten als Substrat für die mikrobiologische Verwertung, die zur Enzymproduktion und zur Erzeugung von Biogas geeignet ist.

Diese Aufgabe wird durch das in den Patentansprüchen beschriebene Verfahren und die in den Patentansprüchen beschriebene Vorrichtung gelöst.

Es hat sich im Rahmen der Erfindung gezeigt, daß frisch extrahierte Zuckerrübenschnitzel in ihrem natürlichen Zustand, also ohne thermische Vorbehandlung, beispielsweise in der Form von Preßlingen, und ähnliche pflanzliche Stückmaterialien, wie beispielsweise bei der Verwertung von Kartoffeln erhaltenes stückförmiges Abfallmaterial, in wirtschaftlicher Weise zur Herstellung von Biomasse verwendet werden können, die ihrerseits zur Enzymproduktion oder zu direkten weiteren Verwertung zur Erzeugung von Biogas (Methanisierung) ausgenutzt werden kann.

Es wurde gefunden, daß Zuckerrübenschnitzel, die beim Auslaugungsverfahren bei der Zuckerherstellung als Abfallprodukt in ausgelaugter Form erhalten werden, mikrobiologisch mit verschiedenen Pilzen, insbesondere Aspergillus niger, in eine wertvolle enzymhaltige Biomasse umgewandelt werden können.

Als besonders geeignet hat sich ein neuer Stamm von Aspergillus niger ergeben, der im folgenden als Aspergillus niger Z 317 bezeichnet wird und bei der deutschen Sammlung von Mikro-Organismen in Göttingen (DSM) hinterlegt wurde. Er hat die Hinterlegungsbezeichnung Z 317 (DSM 3585) erhalten.

Der neue Stamm Aspergillus niger Z 317 läßt sich wie folgt charakterisieren:

Der Schimmelpilz Aspergillus niger Z 317 wurde durch UV-Mutagenese aus einem Wildstamm mutiert und auf Agarplatten mit extrahierten, getrockneten und dann gemahlenen Zuckerrübenschnitzeln selektioniert.

Morphologie

In Submerskulturen aus YEP ( = Yeastextrakt und Pepton) bildet A.niger Z 317 Myzelmasse in Form von Kugeln verschiedener Größe. In Flüssigkulturen mit festen, körnigen Inhaltsstoffen (z.B. Sojabohnenmehl) wächst er vorzugsweise in Kugelform um diese Körner herum.

In Oberflächenkulturen auf Agarplatten mit YEP wächst er zunächst als fädiges Myzel in Kolonien, die sich über die gesamte Platte ausbreiten können. Bei Mangel an Nährstoffen und Feuchtigkeit bildet der Stamm schwarze Konidien aus.

Kulturbedingungen

Der pH-Wert für bestes Wachstum liegt zwischen pH 4, 5 und 5. Die größte Ausbeute an Enzymen wird im Bereich von pH 3,5 bis 4,5 erzielt.

Die für beide Fälle optimale Kulturtemperatur liegt zwischen 23° und 30° C.


Enzym-und Säureproduktion

Aspergillus niger Z 317 liefert folgende Enzyme:
-Pektinasen
-Cellulasen
-Cellobiase
-Hemicellulasen
und überwiegend folgende Säuren:
-Apfelsäure
- Zitronensäure


Enzymcharakterisierung

Die von A. niger Z 317 gebildeten Enzyme zeigen folgende Eigenschaften:

| Enzym | Substrat | c | Aktivit. | $pH_{opt}$ | $T_{opt}$ |
|---|---|---|---|---|---|
| - | - | g/ltr | IU/ml | - | °C |
| Pektinase | Pektin N | 10 | 22 | 4,0 | 50 |
| Pektinase | Pektin C | 10 | 7,4 | 3,5 | 50 |
| Carboxylmethyl-Cellulase | CM-Cellulose | 10 | 5,2 | 4,5 | 55 |
| Cellobiose | Cellobiose | 10 | 0,1 | 4,5 | 60 |
| Xylanase | Xylose | 10 | 3,5 | 3,8 | 45 |

Bei Gemischen aus diesen Substraten und bei natürlichen, komplexen Medien wird ein Enzymkomplex gebildet.


Produktivität

Bei Versuchsfermentationen auf extrahierten Zuckerrübenschnitzeln wurden von A.niger Z 317 folgende Enzymaktivitäten bezogen auf die Schnitzeltrockensubstanz geliefert:

| Enzym | Produktivität |
|---|---|
| - | IU/Kg TS |
| Pektinase | 44.000 |
| Cellulase | 10.400 |
| Cellobiase | 2.000 |
| Xylanase | 7.000 |

Das erfindungsgemäße Verfahren zur Erzeugung von enzymhaltiger Biomasse wird vorteilhaft ausgehend von extrahierten Zuckerrübenschnitzeln durchgeführt. Es läßt sich jedoch auch auf stückförmiges pflanzliches Material anwenden, das Cellulose, Hemicellulose und Pektin enthält. Besonders geeignet sind solche Materialien, die eine ähnliche Zusammensetzung wie die ausgelaugten bzw. extrahierten Zuckerrübenschnitzel aufweisen, die sich in der Trockensubstanz etwa wie folgt zusammensetzen:

Cellulose 24 %
Hemicellulose 10 %
Pektin 24 %
Lignin 6 %
Carbonatasche 7 %
Gesamtstickstoff 3 %
Spuren weitere Stoffe.

Die erfindungsgemäße Verfahren wird vorteilhaft als mehrstufiges Biokonversionsverfahren durchgeführt. Wie oben gezeigt, setzt sich die Trockenmasse der Zuckerrübenschnitzel zum größten Teil aus Pektin, Cellulose und Hemicellulose zusammen. Es hat sich gezeigt, daß Aspergillus niger und insbesondere Aspergillus niger Z 317 die Enzyme Pektinase, Cellulase und Hemicellulase bildet und somit in der Lage ist, auf dem eingesetzten Substrat zu wachsen und dies zu zersetzen. Aspergillus niger und insbesondere Aspergillus niger Z 317 zeichnet sich durch schnelles Wachstum, gute Enzymproduktion und geringe Anfälligkeit gegen Fremdinfektion aus. Außerdem werden Aspergillus niger und deren Stoffwechselprodukte seit langem im allgemeinen als gesundheitlich unbedenklich angesehen.

Wie erwähnt, wird die Erzeugung der enzymhaltigen Biomasse bevorzugt mehrstufig durchgeführt, d.h. das Verfahren wird in ein Anwachsverfahren und eine Enzymproduktionsstufe unterteilt.


1. Die Anwachsstufe

Aus Gründen der Wirtschaftlichkeit wird das Verfahren vorzugsweise unsteril durchgeführt. Das bedeutet, daß während der Anwachsstufe Bedingungen geschaffen werden, unter denen der A. niger einen Selektionsvorteil gegenüber den anderen Organismen hat.

Zur Einleitung des Verfahrens kann so vorgegangen werden, daß A. niger in einer sterilen Vorzucht aus Hefeextrakt oder verdünnter Melasse sicher anwachsen kann. Das gebildete Mycel wird anschließend zerkleinert und mit den Zuckerrübenschnitzeln vermengt. Nun beginnt das Wachstum in der eigentlichen Anwachsstufe ohne Durchmischung auf den Zuckerrübenschnitzeln. Da dies ein aerober Vorgang ist, wird Luft zugeführt.

Die Anwachsstufe wird vorzugsweise in zwei Anwachszonen durchgeführt, in denen jeweils vorzugsweise Temperaturen von 28°C herrschen. Nach einer Aufenthaltszeit von etwa 12 Stunden in der ersten Anwachszone kann ein Teil des bewachsenen Rübenschnitzelsubstrats (beispielsweise die Hälfte) entnommen und der Rest der zweiten Anwachszone zugeführt werden, wo er weitere 12 Stunden bebrütet wird.

Der am Ende der ersten Anwachszone entnommende bewachsene Substratteil wird in einem Mischer mit frischem Substrat und einer Nährlösung vermengt, das auf diese Weise selbsttätig mit Organismen beimpft wird.

Nach Durchlaufen der beiden Anwachsstufen oder -zonen mit einer Verweilzeit von insgesamt 24 Stunden werden die Schnitzel 30 Stunden in der Rührzone durchmischt. Nach Ablauf dieser Zeit ist die maximale Enzymaktivität in der Masse feststellbar. Diese enzymreiche Biomasse wird dann in den nachfolgenden Hydrolysebehälter weitergegeben.

Will man jedoch die Enzyme gewinnen, so werden die Schnitzel 50 Stunden in der Rührzone durchmischt. Die Biomasse ist dann so weit zersetzt, daß die flüssige Phase leicht durch Abpressen gewonnen werden kann. Aus ihr können die gelösten Enzyme beispielsweise durch Sprühtrocknung gewonnen werden.

## 2. Enzymproduktionsstufe

Um eine Konidienbildung zu vermeiden, die der Enzymproduktion entgegensteht, wird das bewachsene Substrat aus der Anwachsstufe (beispielsweise aus der zweiten Zone der Anwachsstufe) in eine Rührzone überführt, wo es unter Bewegen weiter bebrütet wird. Die Temperatur der Rührzone wird bei 28°C gehalten. Die Verweilzeit in der Rührzone beträgt etwa 30 bis 50 Stunden. Die Mischelemente der Rührzone sind so ausgelegt, daß der gesamte Behälterinhalt umgesetzt wird und keine Zonen schlechter Durchmischung entstehen. Vorzugsweise wird auch die Enzymproduktionsstufe in verschiedenen Reaktionszonen mit eigenen Misch-und Luftzufuhrelementen durchgeführt.

Bevorzugt erfolgen die Anwachsphase und die Enzymproduktionsphase in einem vertikalen Behälter, der durch horizontale Trennwände in verschiedene Zonen aufgeteilt wird, wobei das Anwachsverfahren im oberen Teil des Behälters beginnt und das Substrat nach und nach im Laufe des Anwachs-und Enzymproduktionsverfahren jeweils in darunter liegende Zonen überführt wird. Die bei der Kultivierung von A. niger benötigte Atmungs-und Kühlluft wird in einzelnen Zonen beispielsweise über geschlitzte Belüftungsböden zugeführt. Die Abluft aus den einzelnen Zonen wird gesammelt und an die Umgebung, vorzugsweise nach vorherigem Filtern, abgegeben.

Durch die vorstehend aufgezeigte erfindungsgemäße Verfahrensweise wird es möglich, eine Selbstbeimpfung durch Entnahme eines Teils (beispielsweise der Hälfte) des beimpften Materials im Verlaufe der Anwachsstufe durchzuführen. Die Entnahme erfolgt beispielsweise nach einer Inkubationszeit von etwa 12 Stunden. Das entnommene beimpfte Material wird mit frischem unsterilen Substrat (Zuckerrübenschnitzel) und einem Nährmedium vermischt und der Anwachsstufe erneut zugeführt. Bevorzugt wird die Anwachsphase in zwei Zonen durchgeführt, wobei die bewachsene Teilmenge nach der ersten Zone entnommen, der Rest der zweiten Anwachszone zugeführt und das Gemisch aus unsterilem Substrat und beimpften Substrat erneut in die erste Anwachszone eingeführt wird. Bevorzugt wird das unsterile frische Substrat bzw. die ausgelaugten Zuckerrübenschnitzel mit einem flüssigen Nährmedium vermischt, das eine Kohlenstoff-und Stickstoffquelle enthält. Ein derartiges Nährmedium weist beispielsweise 0,5 % Sojabonenmehl und 0,5 % Melasse (bezogen auf W/V = Gewicht pro Volumen) auf, um das Anwachsen zu fördern. Das Nährmedium stellt das Überwachsen des Pilzes von den bereits bewachsenen Schnitzeln auf frische Schnitzel sicher.

Die aus der Rührstufe erhaltene bewachsene enzymhaltige Biomasse kann der weiteren Verarbeitung zugeführt werden.

So ist es möglich, die erhaltene enzymreiche Biomasse in üblicher Weise zur Gewinnung der Enzyme daraus (Cellulase, Hemicellulase, Pektinase) zu verarbeiten. Diese Enzyme können zur weiteren Hydrolyse von frischem Substrat (ausgelaugte Rübenschnitzel) dienen oder beispielsweise bei der Mostherstellung zur Beseitigung von Trübstoffen verwendet werden. Im allgemeinen erfolgt die Isolierung der Enzyme nach dem Auspressen der bewachsenen enzymreichen Biomasse aus der dabei erhaltenen flüssigen Phase.

Eine weitere Möglichkeit zur Verwertung der erhaltenen enzymreichen Biomasse besteht in der direkten Verwertung zur Energiegewinnung bei der Zuckerherstellung. So kann die erhaltene enzymreiche Biomasse direkt zur Hydrolyse von weiterer unsteriler fester Biomasse (ausgelaugte Rübenschnitzel) verwendet und das dabei erhaltene Hydrolysat in üblicher Weise zur Methangewinnung genutzt werden.

Zur Hydrolyse werden die erhaltene Biomasse und frisches Substrat mit Wasser bedeckt und gut durchgemischt Die Hydrolyse erfolgt bei einer Temperatur von 45 bis 50°C und wird beispielsweise während etwa 40 bis 50 Stunden, insbesondere 48 Stunden, durchgeführt. Die Durchmischung erfolgt vorzugsweise mit Rührarmen mit einer Drehgeschwindigkeit von 2 bis 5 UPM. Die Hydrolyse erfolgt bevorzugt in einem vertikalen Hydrolysebehälter mit offenen horizontalen Trennböden, über denen sich Rührarme befinden. Das stückförmige frische Substrat (die ausgelaugten Rübenschnitzel), Wasser und die enzymreiche Biomasse werden von oben in den Behälter eingeführt und durchlaufen durch die Öffnungen der Trennböden die einzelnen Rührzonen des Hydrolysebehälters. Das erhaltene Hydrolysat wird vorzugsweise aus der untersten Stufe entnommen und zur Methanisierungsstufe oder Lagerung weitergeleitet.

Das Gesamtverfahren, Gewinnung der enzymhaltigen Biomasse und anschließende Hydrolyse zur Biogasgewinnung, kann beispielsweise so durchgeführt werden, daß ein Drittel der bei der Zuckerproduktion anfallenden Zuckerrübenschnitzel in der Anwachs-und Enzymproduktionsstufe zu enzymhaltiger Biomasse umgewandelt wird und die restlichen zwei Drittel der angefallenen Zuckerrübenschnitzel in der anschließenden Hydrolysestufe mit der dabei erhaltenen enzymreichen Biomasse hydrolysiert werden.

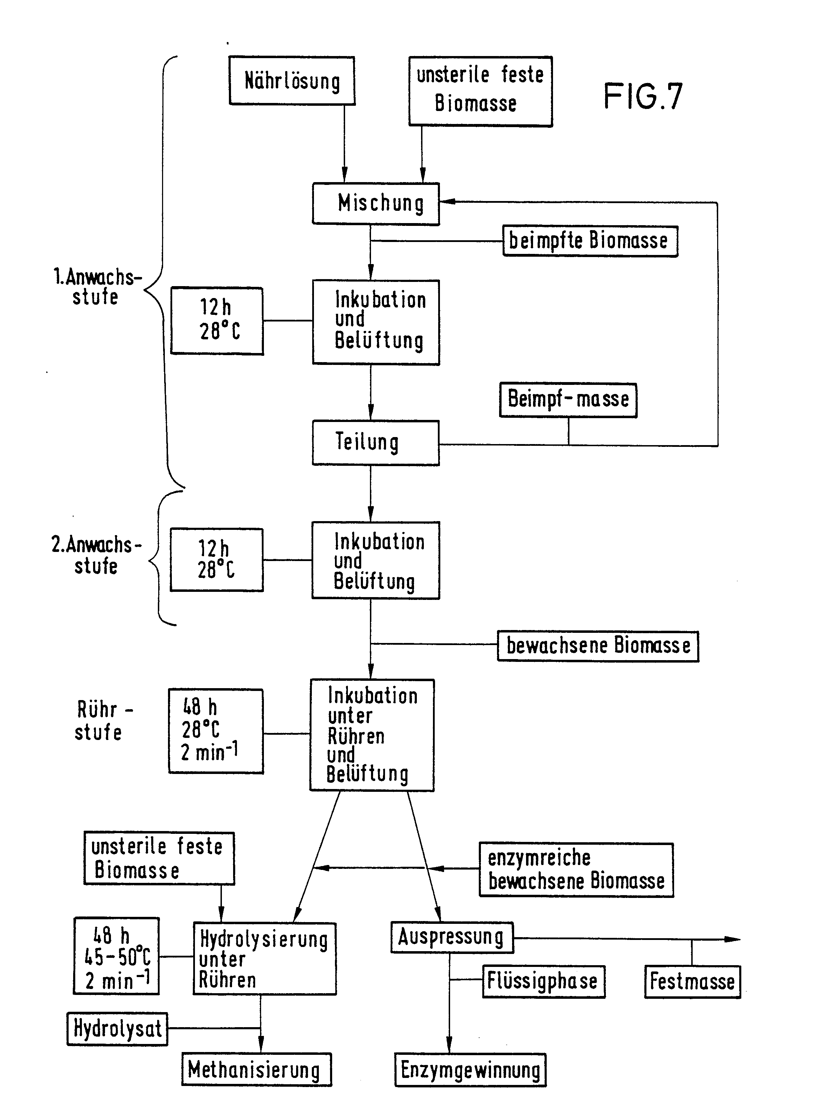

Das als Figur 7 beigefügte Grundfließbild zeigt ein Beispiel für eine erfindungsgemäße Verarbeitung von Zuckerrübenschnitzeln.

Nährlösung (mit einem Gehalt von 0,5 % Sojabohnenmehl und 0,5 % Melasse) wird mit einem Drittel der angefallenen Zuckerrübenschnitzel (unsterile feste Biomasse) vermischt und in einer ersten Anwachsstufe 12 Stunden bei 28°C aerob inkubiert, wobei zur Einleitung des Verfahrens zunächst beimpfte Biomasse (Aspergillus niger, insbesondere Aspergillus niger Z 317) zugesetzt wird. Nach der ersten Anwachsstufe erfolgt eine Teilung, bei der etwa die Hälfte der bewachsenen Zuckerrübenschnitzel als Beimpfmasse zum Mischvorgang zurückgeführt wird. Die nach der Teilung verbleibende Restmasse wird in einer zweiten Anwachsstufe 12 Stunden bei 28°C aerob inkubiert. Die dabei erhaltene bewachsene Biomasse wird dann zur Vermeidung der Konidienbildung in einer Rührstufe etwa 48 Stunden bei 28°C unter weiterem Rühren belüftet. Das Rühren erfolgt mit etwa 2 UPM.

Im Anschluß an das Inkubieren unter Rühren wird die erfindungsgemäß bereitgestellte enzymreiche Biomasse erhalten.

Die erhaltene Biomasse kann zwei Wegen weiterverarbeitet werden. In dem Beispiel der Figur 7 werden weitere Rübenschnitzel (die restlichen zwei Drittel der angefallenen unsterilen festen Biomasse) mit der Biomasse unter Rühren unter Bedeckung mit Wasser 48 Stunden bei 45 bis 50°C hydrolysiert. Das Rühren erfolgt mit 2 UPM. Das erhaltene Hydrolysat kann der üblichen Methanisierung zugeführt werden.

Als weitere Alternative bietet sich die Möglichkeit, die enzymreiche hydrolysierte Biomasse durch Auspressen in eine Festmasse und eine Flüssigphase aufzutrennen und aus der Flüssigphase die Enzyme Pektinase, Cellulase und Hemicellulase zu gewinnen.

Alternativ kann die enzymhaltige Flüssigphase auch zur Hydrolyse weiterer Zuckerrübenschnitzel - (beispielsweise der restlichen zwei Drittel der nicht bewachsenen unsterilen festen Biomasse) verwendet werden. Hierbei wird die gesamte enzymreiche Biomasse, d.h. also die feste und die flüssige Phase, in den Hydrolysebehälter gegeben, wo sie weiteres Substrat zersetzt.

Eine Hydrolyse ist aber auch mit der Enzymlösung allein, also ausschließlich mit der flüssigen Phase, möglich. Dann geht jedoch ein Teil der Enzyme mit der festen Phase verloren.

Die Erfindung betrifft auch eine Vorrichtung, die zur Durchführung des erfindungsgemäßen Verfahrens, d.h. zur Herstellung von enzymhaltiger Biomasse und gegebenenfalls zur weiteren Gewinnung von Enzymen und/oder Biogas geeignet ist. Die Vorrichtung und insbesondere der Bioreaktor der Vorrichtung eignet sich jedoch auch allgemein zur mikrobiologischen Hydrolyse und zur kontinuierlichen Kultivierung von Mikroorganismen auf stückförmigen Nährstoffen, insbesondere pektin-, cellulose-und hemicellulosehaltigen landwirtschaftlichen Abfällen, wie beispielsweise den vorstehend beschriebenen extrahierten Zuckerrübenschnitzeln, stückförmigen Kartoffelabfallprodukten oder anderen stückförmigen pflanzlichen Abfallprodukten. Die im Bioreaktor der erfindungsgemäßen Vorrichtung erhaltene bewachsene Biomasse kann beispielsweise zur Herstellung von Enzymen verwendet oder zur enzymatischen Hydrolyse weiterer pflanzlicher Abfallprodukte, beispielsweise zum Zwecke der anschließenden Methanisierung, eingesetzt werden.

Die Vorrichtung besteht aus einem bevorzugt zylindrischen ersten vertikal aufgestellten geschlossenen Behälter, der durch mit durch Klappen verschließbaren Öffnungen versehene horizontale Trennböden in eine obere, eine mittlere und eine untere Zone aufgeteilt wird. Die obere Zone dient als erste Anwachszone, die mittlere Zone dient als zweite Anwachszone und die untere Zone dient zur weiteren Inkubation unter Bildung der gewünschten bewachsenen Biomasse (beispielsweise der enzymhaltigen bewachsenen Zuckerrübenschnitzel). Sie ist mit Mischelementen bzw. Rührwerken ausgerüstet und wird daher im folgenden als Rührstufe bezeichnet. Die erste und zweite Anwachszone weisen keine Mischelemente auf, um ein möglichst bewegungsfreies und ungestörtes Anwachsen der Mikroorganismen, beispielsweise der Mycelien, zu ermöglichen.

Die drei Zonen des Bioreaktors sind bevorzugt jeweils durch weitere Trennböden mit durch Klappen verschließbaren Öffnungen in verschiedene Zonen unterteilt. So wird beispielsweise die erste Anwachszone durch zwei bis drei Trennböden in drei bis vier gleich große Kammern unterteilt.

Die zweite Anwachszone wird beispielsweise durch ein oder zwei Trennböden in zwei bis drei gleich große Kammern unterteilt, und die Rührzone wird beispielsweise durch fünf bis sechs Trennböden in sechs bis sieben gleich große Zonen unterteilt.

Gemäß einer bevorzugten Ausführungsform des erfindungsgemäßen Bioreaktors weisen die erste Anwachszone drei Kammern, die zweite Anwachszone zwei Kammern und die Rührzone sechs Kammern auf.

Der Bioreaktor stellt vorzugsweise ein zylinderförmiges Gefäß dar, das an seinem oberen und unteren Ende geschlossen ist. In diesem Falle sind die Trennböden kreisförmig ausgebildet und liegen an der Innenwand des zylinderförmigen Behälters an. Die Trennböden weisen eine oder mehrere Öffnungen auf, durch die das Beschickungsgut der einzelnen Kammern bzw. Zonen in die nächstuntere Kammer bzw. Zone einfallen kann. Das Beschickungsgut wird hierzu durch von außen steuerbare bewegliche Ausräumer zu den Öffnungen geschoben. Derartige von außen steuerbare Ausräumer können beispielsweise Rührarme sein, die sich von einer zentralen Welle im Inneren der zylinderförmigen Kammer radial nach außen erstrecken. Beispielsweise ist in den Kammern der Anwachszonen der Ausräumer in der Form eines Stabes bzw. Balkens ausgebildet, der etwas kürzer als der Innendurchmesser des zylinderförmigen Gefäßes ist und durch dessen Mitte eine zentrale Antriebswelle läuft. In den Kammern der Anwachszonen sind die Ausräumerarme so ausgebildet, daß eine übermäßige Rührwirkung möglichst vermieden wird. Die Ausräumer der Kammern der Rührzone hingegen sind in der Form von Mischelementen ausgeführt, die ein Rührprofil aufweisen, das zur Durchmischung der Biomasse geeignet ist. Derartige, gleichzeitig zum Ausräumen geeignete Mischelemente sind beispielsweise an einer zentralen Welle befindliche Rührarme mit keilförmigem Rührprofil, durch die sich nach oben und unten herausragende Querstäbe erstrecken.

Über den Ausräumern sind in den einzelnen Kammern der Anwachszonen sowie der Rührzone jeweils Belüftungsrohre angebracht, die bei kreisförmigem Querschnitt des Bioreaktors diesen z.B. kreisförmig gebogen oder in Form von zwei Halbkreisen, in jedem Falle parallel zu den Trennböden, durchlaufen. Die Belüftungsrohre weisen zahlreiche zum Luftaustritt geeignete Öffnungen auf, die sich vorzugsweise an der Unterseite der Belüftungsrohre befinden. Über den Belüftungsrohren der einzelnen Kammern der Anwachszonen kann die Biomasse im wesentlichen unbewegt, durchströmt von Luft, ohne Störung beispielsweise einer Mycelbildung, ruhen.

Im Gegensatz hierzu weisen die einzelnen Kammern der Rührzone vorzugsweise ein zweites oberes Mischelement auf, das über den Belüftungsrohren z.B. an einer die Kammer durchlaufenden zentralen Welle angebracht ist. Das obere Mischelement ist im Prinzip genauso ausgebildet wie das untere Mischelement, das gleichzeitig als Ausräumer dient. Es besteht im allgemeinen aus einem Rührbalken mit keilförmigem Rührprofil, das von mehreren senkrechten Querstäben durchsetzt ist, die eine gute Durchmischung des Rührgutes sichern. Da der über den Belüftungsrohren liegende Teil der Kammern höher ist als der unter den Belüftungsrohren liegende Teil, der lediglich dem Ausräumen der Kammern dient, ist das obere Mischelement im allgemeinen kräftiger bzw. größer ausgebildet als das untere Mischelement und weist längere Querstäbe auf.

Die Mischelemente und Ausräumer der einzelnen Kammern können unabhängig voneinander gesteuert werden. Nach einer bevorzugten Ausführungsform der Erfindung ist der zylinderförmige Bioreaktor jedoch von einer zentralen Welle durchsetzt, an die die einzelnen Mischelemente und Ausräumer angebracht sind oder angekoppelt werden können. Dies hat den Vorteil, daß die zentrale Welle von einem einzigen Antriebsmotor angetrieben werden kann.

Die Trennböden zwischen den einzelnen Kammern und Zonen weisen eine oder mehrere Öffnungen auf. Vorzugsweise sind diese Öffnungen als rechteckige Spalte ausgebildet, die sich in ihrer Längsrichtung vorzugsweise radial erstrecken. Die Öffnungen sind durch bevorzugt rechteckige Fallklappen verschließbar, die durch einen von der Außenseite des Bioreaktors angelenkten Steuerarm betätigt werden können. Alternativ werden die Fallklappen bei Überfüllung der darunter liegenden Kammer durch das Füllgut automatisch geschlossen. Die Fallklappen dienen außer zum Verschließen der Öffnungen der Trennböden auch zur Steuerung des Substratdurchsatzes. Durch verschieden große Öffnungswinkel wird mehr oder weniger Substrat in die nächste Kammer befördert. Der Öffnungswinkel wird durch den Füllgrad der darunterliegenden Kammer bestimmt. Der Füllstand einer Kammer wird beispielsweise induktiv aufgenommen und als Stellwert an die Fallklappe des darüberliegenden Trennbodens weitergegeben, die sich somit nach Bedarf weiter öffnet oder schließt.

Die Zufuhr an frischen Schnitzeln wird durch die Viskosität in der untersten Rührkammer bestimmt. Ist ein gewünschter Zersetzungsgrad dort nicht erreicht, so wird in die erste Anwachszone weniger frisches Substrat eingefüllt. Dadurch wird die Verweilzeit im Reaktor so weit verlängert, bis sich die gewünschte Viskosität wieder einstellt. Umgekehrt kann auch bei Überschreiten der gewünschten Viskosität die Zugabe an Substrat erhöht werden. Die Totzeit der Steuerung beträgt je nach Aufenthaltszeit 45 bis 72 Stunden.

Am oberen Ende der einzelnen Kammern befinden sich vorzugsweise Abluftrohre, durch die die mittels der Belüftungsrohre eingeführte Luft in eine gemeinsame Abluftrohrleitung entweichen kann. Die den einzelnen Kammern bzw. Zonen zuzuführende Luftmenge wird von einem Thermofühler bestimmt, der an einer repräsentativen Stelle im Füllbereich jeder Kammer angebracht ist. Übersteigt die Substrattemperatur den gewünschten Sollwert, so erhöht ein angesteuertes Drosselventil die dieser Kammer zugeführte Luftmenge. Bei Unterschreiten der Solltemperatur wird die Luftmenge entsprechend gedrosselt.

Jede Kammer wird separat be-und entlüftet. Das hat die vorstehend erwähnten Vorteile bei der Temperatursteuerung.

Am oberen Ende der obersten Kammer der ersten Anwachszone befindet sich eine Beschickungsöffnung, die von einer Mischeinrichtung mit der zu verarbeitenden Biomasse beschickt werden kann. Diese dem Bioreaktor vorgeschaltete Mischeinrichtung kann in Form einer Mischschnecke in den Bioreaktor integriert sein. Eine derartige Mischschnecke stellt eine übliche Misch-bzw. Fördereinrichtung dar, die nicht näher erläutert werden muß. Sie weist einen Zufuhrtrichter für die aufzugebenden Ausgangsmaterialien (beispielsweise Zuckerrübenschnitzel und Nährlösung) auf. Darüber hinaus weist sie einen weiteren Eingang für Beschickungsmaterialien auf, der in Verbindung mit einer Fördereinrichtung steht - (beispielsweise einem Steilförderband), die ihrerseits durch eine Beschickungseinrichtung gespeist wird, die in das obere Ende der obersten Kammer der zweiten Anwachsstufe des Bioreaktors so eingebaut ist, daß sie einen Teil des aus der Öffnung bzw. den Öffnungen des unteren Trennbodens der ersten Anwachszone fallenden beimpften Produkts aus dem Bioreaktor heraustransportieren und in die Steilfördereinrichtung einführen kann. Der erfindungsgemäße Bioreaktor ist somit mit einer Einrichtung versehen, die die Entnahme einer Teilmenge des in der ersten Anwachszone erhaltenen beimpften Produkts und deren Einführung in die Mischzonen zur Vermischung mit dem Beschikkungsgut für die erste Anwachszone ermöglicht. Durch den erfindungsgemäßen Reaktor wird somit eine Selbstbeimpfung des mikrobiologisch zu verarbeitenden Beschickungsgutes, beispielsweise der Zuckerrübenschnitzel, in kontinuierlicher Weise möglich.

Dem ersten Behälter (Bioreaktor) kann sich ein zweiter vertikal aufgestellter Behälter - (Hydrolysebehälter) anschließen. Dieser Behälter ist vorzugsweise zylindrisch. Er ist oben und unten verschlossen und durch offene Trennböden in mehrere Kammern (beispielsweise in vier bis zehn Kammern) unterteilt. Jede Kammer ist mit einem Rührsystem ausgerüstet, das eine gute Durchmischung des Gutes gewährleistet. Bevorzugt werden sämtliche Rührer von einer zentralen Welle gemeinsam angetrieben. Die Rührer können wie beim Bioreaktor ausgebildet sein, beispielsweise als keilförmige oder winkelförmige Rührarme, die von senkrechten Querstäben durchsetzt sind. Jede einzelne Kammer weist einen oder mehrere Rührer auf.

Am oberen Ende des Hydrolysebehälters befinden sich ein Einlaß für frische Biomasse (beispielsweise Zuckerrübenschnitzel) sowie ein Einlaß für den Zulauf von enzymhaltiger Biomasse oder enzymhaltiger Lösung, die aus der enzymhaltigen Biomasse gewonnen wurde. Am unteren Ende des Gefäßes befindet sich ein Auslaß für das erhaltene Produkt (Hydrolysat). Der Behälter kann am oberen Ende einen weiteren Einlaß für die Zufuhr von Wasser aufweisen. Das Wasser kann jedoch auch durch den Einlaß für die frische Biomasse oder durch den Einlaß für die Enzymlösung eingeführt werden. Der Behälter ist so konstruiert, daß seine Wand zur Wärmeisolierung und/oder Temperierung des enthaltenen Substrats geeignet ist.

In der Trockenstufe der Vorrichtung werden etwa 30 % und in der Flüssigstufe etwa 70 % des Substrats, beispielsweise der Zuckerrübenschnitzel, zugegeben. Die Verweilzeit in der Trockenstufe bzw. Trockenkolonne beträgt für eine Enzymgewinnung etwa 72 Stunden. Am Ende der Kolonne wird aus dieser eine pastöse Enzym-Masse abgezogen, die anschließend noch hydrolysiert werden kann.

Durch die Erfindung ist eine kontinuierliche Arbeitsweise mit selbsttätiger Beimpfung ohne Fremdbeimpfung möglich. Die erfindungsgemäße Vorrichtung bzw. Anlage läßt sich äußerst kompakt und damit raumsparend bauen. Sie kann mit hohem Füllgrad von etwa 90 % und sogar mehr betrieben werden. In den einzelnen Kammern ist wegen der eingebauten Mischelemente bzw. Rührwerke eine hohe Packungshöhe von beispielsweise etwa 50 bis 80 cm möglich. Der Fall-Transport des Gutes von Kammer zu Kammer der Vorrichtung ermöglicht eine $O_2$-Zufuhr über die Oberfläche des Substrates bzw. Gutes.

Mit der Vorrichtung bzw. Anlage kann man auch Hefepilze, Bakterien und auch anaerobe Bakterien züchten.

In der Zeichnung ist ein Ausführungsbeispiel einer erfindungsgemäßen Vorrichtung zum Durchführen des erfindungsgemäßen Verfahrens schematisch dargestellt, und zwar zeigt

Fig. 1 einen senkrechten Schnitt der Vorrichtung,
Fig. 2 einen senkrechten Schnitt ähnlich wie in Fig. 1 eines Hydrolysebehälters der Vorrichtung,
Fig. 3 eine Draufsicht auf einen Trennboden, woraus die Fallöffnungen zu ersehen sind,
Fig. 4 einen senkrechten Teilschnitt eines Trennbodens in gegenüber Fig. 1 bis 3 größerem Maßstab,

Fig. 5 einen Querschnitt der Vorrichtung nach Linie V -V aus Fig. 4,

Fig. 6 einen Teilschnitt eines Rührwerkes nach Linie VI -VI aus Fig. 5 in nochmals vergrößertem Maßstab, woraus eine Ausführungsform des Querschnittes eines Rührarmes zu erkennen ist,

Fig. 7 ein Fließbild des erfindungsgemäßen Verfahrens.

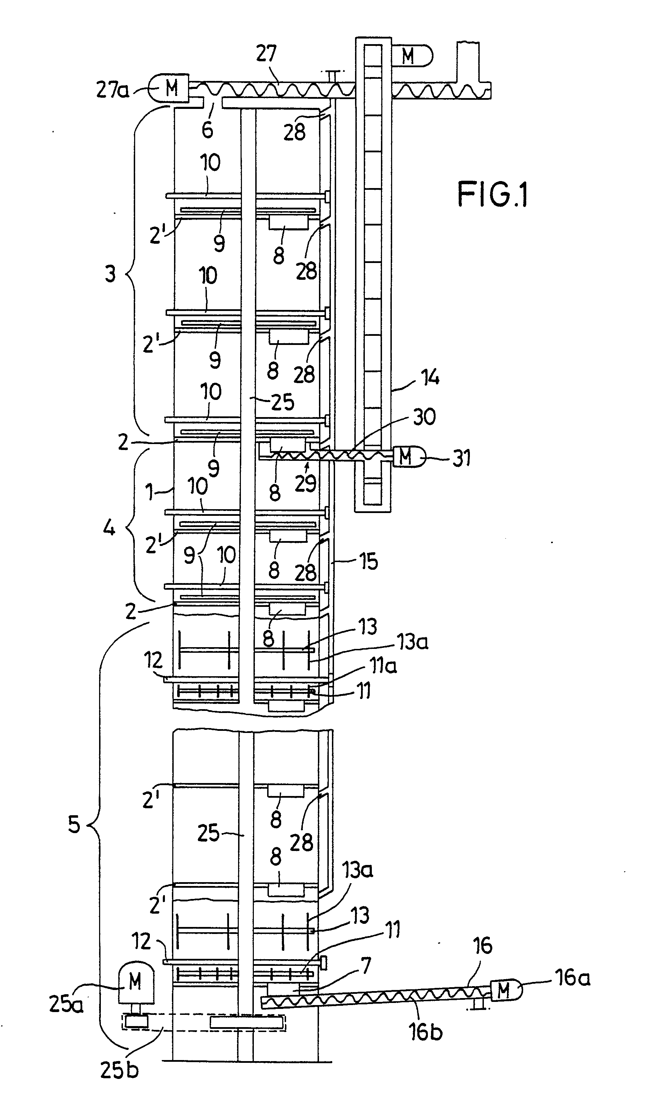

Die in Fig. 1 gezeigte Vorrichtung, welche als Bioreaktor eingesetzt werden kann, weist einen zylinderförmigen Behälter 1 auf, der in zwei Anwachszonen 3 und 4 und eine Rührzone 5 durch vertikale Trennböden 2 unterteilt ist. Frisches Substrat (Zuckerrübenschnitzel, gegebenenfalls plus Nährlösung) wird über eine Fördereinrichtung 27, die in dem speziellen Beispiel eine von einem Motor 27 a angetriebene Förderschnecke aufweist, durch einen Einlaß 6 in eine erste Kammer der ersten Anwachszone 3 eingeführt. Diese Kammer ist durch einen glatten horizontalen Trennboden 2' von der darunter liegenden zweiten Kammer der Anwachszone 3 getrennt. An einer zentralen Welle 25 ist ein Ausräumer 9 angebracht, der das Substrat zu Öffnungen im Trennboden, die jeweils durch eine Fallklappe bzw. Bodenklappe 8 verschlossen werden können, bewegt. Durch ein Rohr 10, das über dem Ausräumer 9 angeordnet ist und Austrittsöffnungen enthält, wird dem in dieser Kammer befindlichen Gut in ausreichender Menge notwendige Kühlund Atmungsluft zugeführt. Die Abluft kann durch Öffnungen 28 in eine Abluftleitung 15 entweichen.

Die Anwachszone 3 ist durch einen weiteren Zwischenboden 2' in zwei weitere Kammern unterteilt, deren Bauweise der ersten Kammer entspricht. Das zu bewachsende Substrat verweilt in den einzelnen Kammern der Anwachszone 3 im wesentlichen ohne Bewegung. Es wird lediglich durch die Ausräumer 9 zu den Bodenöffnungen geführt und fällt bei geöffneter Klappe 8 in die darunter liegende Kammer, wo es weiter unbewegt verbleibt. Die Fallklappen 8 sind von außen steuerbar. Sie werden durch das in der darunter liegenden Kammer befindliche Gut automatisch geschlossen, wenn in dieser Kammer eine gewisse Füllhöhe erreicht worden ist.

Unter dem Trennboden 2 der letzten Kammer der Anwachszone 3 ist eine Fördereinrichtung 29 mit einer Förderschnecke 30 und einem Antriebsmotor 31 vorgesehen, die Teile des austretenden bewachsenen Substrats über ein Steilförderband 14 zur Fördereinrichtung 27 zurückführen kann. Hierdurch wird eine Selbstanimpfung des von der Fördereinrichtung 27 zugeführten Substrats erzielt.

Der Rest des beimpften Substrats tritt in eine erste obere Kammer der zweiten Anwachszone 4 ein und gelangt von dieser in eine darunter liegende zweite Kammer. Die Kammern der zweiten Anwachszone 4 weisen die gleiche Bauweise wie die der ersten Anwachszone 3 auf.

Anschließend tritt das bewachsene Substrat durch Öffnungen im Trennboden 2 der unteren Anwachszone 4 in die erste oberste Kammer der Rührzone 5 ein. Die Kammern der Rührzone 5 weisen im Prinzip eine ähnliche Bauweise wie die Kammern der Anwachszonen 3 und 4 auf. Lediglich die Ausräumer 11 sind hier als Rührarme ausgebildet, die Querstäbe 11a aufweisen und ein gutes Durchmischen des Substrats ermöglichen. Ferner befinden sich über Luftzufuhrrohren 12 die Rührer 13, die zur guten Durchmischung des Rührgutes ebenfalls Querstäbe 13a aufweisen. Die Rührzone 5 ist in mehrere Kammern gleicher Bauweise unterteilt. Die Austrittsöffnung im Boden der letzten Kammer der Rührzone 5 ist als Produktauslaß 7 ausgebildet, der ebenfalls mit einer Fallklappe verschließbar ist. An die Austrittsöffnung ist eine Fördereinrichtung 16 angeschlossen, die mit einem Motor 16a versehen ist und beim dargestellten Ausführungsbeispiel eine Förderschnecke 16b aufweist.

Ein Motor 25a am unteren Ende des Behälters 1 dient zum Antrieb der zentralen Welle 25 und damit der an dieser angebrachten Ausräumer und Rühreinrichtungen. Der Motor 25a ist vorzugsweise als Getriebemotor ausgebildet und treibt über eine Transmission wie einen Kettentrieb 25b die zentrale Welle 25 mit einer Drehzahl von 2 bis 5 Upm an.

Die Geometrie der Rührarme in den einzelnen Kammern der Rührstufe kann gleich sein, jedoch können ihre Abmessungen, ihre Anstellung, ihre Anzahl und die Abmessungen und Anzahl der Querstäbe so gewählt sein, daß sie entsprechend dem jeweilegen Rührgut überall eine gleich gute Durchmischung bewirken.

Die Fördereinrichtung 27, die zum Produkteinlaß 6 am oberen Ende des Behälters führt, kann eine Mischeinrichtung sein, die zum Vermischen von Rübenschnitzeln wie Preßschnitzel mit weiterem Material - (beispielsweise im Mischungsverhältnis 1:1) und mit den aus der ersten Anwachszone 3 abgezogenen Impfschnitzeln geeignet ist.

Die Fördereinrichtung 29 am Ende der ersten Anwachszone 3 kann eine beliebige Fördereinrichtung sein. Sie enthält hier eine Förderschnecke 30, die mit verstellbarer Drehzahl angetrieben werden kann, so daß etwa 50 % der aus der ersten Anwachszone 3 austretenden beimpften Zuckerrübenschnitzel abgezogen werden können.

Anstelle des Steilförderbandes 14 und der Fördereinrichtung 16 können auch Pumpen mit entsprechenden Rohrleitungen vorgesehen sein.

9

Die Trennböden 2 und 2' dienen zur Begrenzung der Schichtdicke des Substrats in den einzelnen Kammern. Sie sind glatt ausgebildet, so daß Substrat leicht durch die Ausräumer bewegt werden kann.

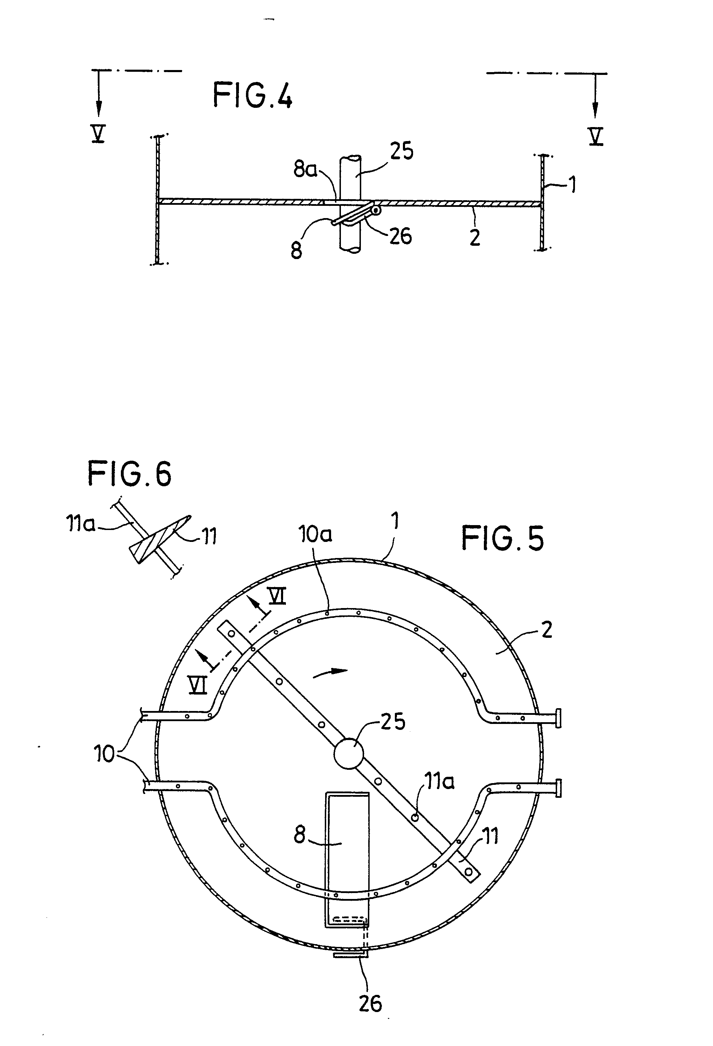

Die Fig. 4 und 5 zeigen Ausführungsformen von kreisförmigen Trennböden für einen zylindrischen Behälter 1. Sie sind von der zentralen Antreibswelle 25 durchsetzt und enthalten rechteckige, in ihrer Längsrichtung radial angeordnete Öffnungen 8a, die durch entsprechende rechteckige Fallklappen 8 verschlossen werden können. Wenn das in der unter einem Trennboden liegenden Kammer befindliche Gut eine gewisse Füllhöhe erreicht, wird die Fallklappe 8 von dem in Bewegung befindlichen Gut hochgedrückt und damit geschlossen. Außerdem ist die Fallklappe 8 durch einen Steuerarm 26, der von der Außenseite des Behälters 1 betätigt werden kann, steuerbar und verschließbar. In der Fig. 5 sind auch die Luftzufuhrrohre 10 bzw. 12 zu erkennen. Diese enthalten Löcher 10a, die bevorzugt an der Unterseite liegen. In Fig. 5 ist ein Ausräumer 11 mit Querstäben 11a dargestellt. Die weiteren Ausräumer 9 und die Rührarme 13 sind ähnlich angeordnet und/oder ausgebildet.

Fig. 6 zeigt den Querschnitt durch einen Ausräumer 11 mit keilförmigem Rührprofil und Querstäben 11a.

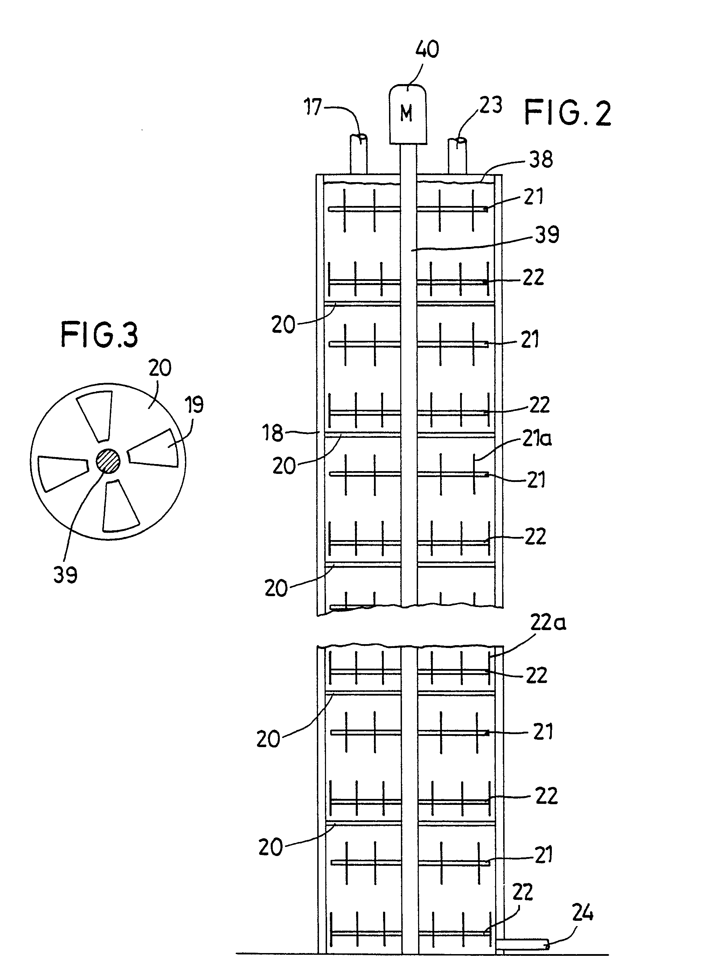

Ein Beispiel für den Hydrolysebehälter 18 ist in Fig. 2 dargestellt. Durch eine Einlaßöffnung 17 kann mittels Förder-bzw. Pumpeinrichtungen Biomasse aus dem Behälter 1 oder aus dieser gewonnene Enzymlösung am oberen Ende in den Behälter 18 eintreten. Frisches Substrat (Rübenschnitzel) kann durch eine weitere Einlaßöffnung 23 ebenfalls am oberen Ende in den Behälter 18 eintreten. Wasser kann durch die Einlaßöffnungen 17 oder 23 zugeführt werden.

Der Hydrolysebehälter 18 ist durch Trennböden 20 mit segmentförmigen Öffnungen 19 in mehrere Kammern gleicher Bauweise aufgeteilt. In jeder Kammer befinden sich zwei Rührer 21 und 22, die jeweils mit Querstäben 21a bzw. 22a versehen sind.

Das zu hydrolisierende Gut durchläuft den Hydrolysebehälter 18 vertikal von oben nach unten. Die Wasserhöhe wird so gehalten, daß das gesamte Beschickungsgut bedeckt ist (Wasserspiegel 38). Das Gut wird beim Durchlaufen der einzelnen Kammern ständig durch die Rührer 21 und 22 bewegt, welche von einer zentralen Welle 39 mit Antriebsmotor 40 angetrieben werden. Das Hydrolysat tritt schließlich durch einen Auslaß 24 aus dem Hydrolysebehälter 18 zur weiteren Verarbeitung (z.B. Methanisierung) oder Lagerung aus.

Fig. 3 zeigt einen kreisförmigen Trennboden 20 mit segmentförmigen Öffnungen 19.

Die Größe des Hydrolysebehälters 18 ist so bemessen, daß für die Mazeration der zu hydrolysierenden frischen Biomasse eine Verweilzeit von etwa 48 Stunden gewährleistet ist.

Die Abmessungen des Bioreaktors sind so gewählt, daß in der ersten Anwachszone eine Verweilzeit von 12 Stunden, in der zweiten Anwachszone eine Verweilzeit von nochmals 12 Stunden und in der Rührzone eine Verweilzeit von 48 Stunden gewährleistet ist.

Die Belüftung des Bioreaktors erfolgt mit Umgebungsluft. Die Steuerung des Luftdurchsatzes kann über einen Thermofühler erfolgen, der die Temperatur des zu behandelnden Gutes auf dem gewünschten Wert von 28°C hält und maximal auf 34°C begrenzt.

Die Steuerung der Substratzugabe in den Bioreaktor kann durch Messung der Viskosität des Gutes in der untersten Stufe erfolgen. Nach dem Mazerationsgrad ist eine Aussage über die gebildete Enzymmenge möglich.

Die vorstehend angegebene Inkubationszeit von etwa 48 Stunden in der Rührzone des Bioreaktors ist dann besonders günstig, wenn die gesamte erhaltene enzymreiche Biomasse zur Mazeration von frischem Substrat in der Hydrolysestufe verwendet werden soll. Längere Inkubationszeiten von bis zu 72 Stunden können jedoch günstig sein, wenn die Enzyme als solche aus der flüssigen Phase der enzymhaltigen Biomasse gewonnen werden sollen. Die Größe des Bioreaktors wird für diesen Fall entsprechend ausgelegt.

Mit der Vorrichtung ist eine günstige Verwertung von in der Zuckerindustrie abfallenden extrahierten Zuckerrübenschnitzeln möglich. Eine derartige Verwertung war bisher nicht möglich. Sie ist besonders vorteilhaft, da in der Zuckerindustrie große Mengen an derartigen Abfallprodukten anfallen. Beispielsweise fallen in einer Zuckerfabrik mit einer Schneidleistung von 5000 Tagestonnen pro Stunde 45,8 Tonnen Zuckerrübenschnitzel als Preßlinge mit durchschnittlich 22 % Trockensubstanz an. Das entspricht einer Trockenmasse von 10,1 to/h. Das Schüttgewicht liegt zwischen 500 und 600 kg/m³, und die Temperatur beträgt 42°C. Der Energiebedarf eines Herstellers von Zucker-Grundsorte liegt zwischen 0,8 und 0,9 GJ/to Rüben. Das sind ca. 180 GJ/h. Davon müssen für die Trocknung der Preßlinge 5 bis 5,8 GJ/to Trockengut = 60 GJ/h aufgebracht werden. Ohne Trocknung benötigt eine Zuckerfabrik der vorstehenden Größe daher etwa 120 GJ/h = 3,3 to Öl/h. Es hat sich gezeigt, daß dieser Energiebedarf durch die erfindungsgemäße Methanisierung der Zuckerrübenschnitzel bereits zur Hälfte gedeckt werden kann, wenn lediglich von einer 50 %-igen Ausnutzung der Schnitzel ausgegangen wird.

10

## Ansprüche

1. Verfahren zum Herstellen enzymhaltiger Biomasse, die zur Gewinnung von Enzymen und Biogas geeignet ist, durch mikrobiologische Behandlung von bei der Zuckerherstellung anfallenden Abfallprodukten, dadurch **gekennzeichnet,** daß ausgelaugte Zuckerrübenschnitzel mit Aspergillus niger fermentiert werden.

2. Verfahren nach Anspruch 1, dadurch **gekennzeichnet,** daß mit Aspergillus niger - (Hinterlegungsbezeichnung Z 317 -DSM 3585) fermentiert wird.

3. Verfahren nach Anspruch 1 oder 2, dadurch **gekennzeichnet,**daß man

a) ausgelaugte Zuckerrübenschnitzel mit einem flüssigen Nährmedium, das eine Kohlenstoff-und Stickstoffquelle enthält, tränkt, mit mit Aspergillus niger beimpfter Biomasse vermischt,

b) das erhaltene Gemisch in einer ersten bewegungsfreien Anwachsstufe unter Belüften inkubiert,

c) von der in b) erhaltenen Biomasse einen Teil abtrennt und als beimpfte Biomasse zur Mischstufe a) zurückführt, und den Rest

d) in einer zweiten bewegungsfreien Anwachsstufe unter Belüften inkubiert,

e) die in d) erhaltene bewachsene Biomasse einer weiteren aeroben Inkubation unter ständigem Bewegen der Biomasse unterzieht, und

f) gegebenenfalls die in e) erhaltene enzymreiche Biomasse mit frischen ausgelaugten Zuckerrübenschnitzeln und Wasser versetzt und unter Bewegen hydrolysiert, das gewonnene Hydrolysat von der festen Phase abtrennt und einer üblichen anaerob arbeitenden Methanisierungsstufe unter Bildung von Biogas zuführt, oder

g) gegebenenfalls aus der in e) erhaltenen enzymreichen Biomasse die darin enthaltenen Enzyme in üblicher Weise isoliert.

4. Vorrichtung zum Durchführen des Verfahrens nach einem der Ansprüche 1 bis 3, dadurch **gekennzeichnet,** daß sie einen geschlossenen vertikalen Behälter (1) aufweist, der durch horizontale Trennböden - (2) in eine obere erste Anwachszone (3), eine mittlere zweite Anwachszone (4) und eine untere Rührzone (5) unterteilt ist, einen Beschickungseinlaß (6) im oberen Teil der ersten Anwachszone (3) und einen Produktauslaß (7) im unteren Teil der Rührzone (5) hat und jeweils wenigstens einen horizontalen Trennboden (2') innerhalb der Anwachszonen (3,4) und der Rührzone (5) enthält, wobei die Trennböden - (2,2') mit jeweils wenigstens einer durch Fallklappen (8) verschließbaren Öffnung versehen, über den Trennböden (2,2') der Anwachszonen (3,4) bewegliche Ausräumer (9) angeordnet und über diesen liegende, mit rohrförmigen Öffnungen versehene Luftzufuhrrohre (10) vorgesehen sind, ferner über den Trennböden (2,2') der Rührzone (5) als Ausräumer ausgebildete Mischelemente (11), über diesen Luftzufuhrrohre (12) und über letzteren weitere Mischelemente (13) sowie eine Rückführeinrichtung (14), die zum Rückführen eines Teils des aus dem unteren Trennboden (2) der ersten Anwachszone (3) austretenden Produkts zum Beschickungseinlaß (6) oder in eine vor diesem liegende Mischzone für das Beschickungsgut geeignet ist, und Abluftleitungen (15) in jeder der Zonen.

5. Vorrichtung nach Anspruch 4, dadurch **gekennzeichnet,** daß eine Fördereinrichtung (16) den Produktauslaß (7) mit dem Einlaß (17) am oberen Ende eines zweiten geschlossenen vertikalen Behälters - (18) verbindet, der durch mit Öffnungen (19) versehene horizontale Trennböden (20) in mehrere mit Rührern (21,22) ausgerüstete Kammern unterteilt ist und an seinem oberen Ende einen weiteren Beschickungseinlaß (23) und an seinem unteren Ende einen Produktauslaß (24) aufweist.

6. Vorrichtung nach Anspruch 4 oder 5, dadurch **gekennzeichnet,** daß die Ausräumer (9) und die Mischelemente (11,12) an einer zentralen Antriebswelle (25) angeordnet sind.

7. Vorrichtung nach einem der Ansprüche 4 bis 6, dadurch **gekennzeichnet,** daß die Luftzufuhrrohre - (10) an ihrer Unterseite mit Löchern versehen sind.

8. Vorrichtung nach einem der Ansprüche 4 bis 7, dadurch **gekennzeichnet,** daß die Fallöffnungen spaltförmig in ihrer Längsrichtung radial zum betreffenden Trennboden (2,2') verlaufend ausgebildet sind.

9. Vorrichtung nach Anspruch 8, dadurch **gekennzeichnet,** daß die Fallklappen (8) durch einen Steuerarm (26) zu betätigen sind, der außerhalb des Behälters (1) angelenkt ist.

FIG.1

FIG.2

FIG.3

# FIG.4

25
8a
1
8
26
2

# FIG.6

11a
11

# FIG.5

10a
1
2
VI
VI
10
25
11a
8
11
26

FIG.7